# EUROPEAN PATENT SPECIFICATION

(11) **EP 1 847 256 B1**
(45) Date of publication and mention of the grant of the patent: **23.04.2014**
(21) Application number: 07007272.3
(22) Date of filing: 06.04.2007
(51) Int. Cl.: A61K 9/12, A61K 31/496, A61K 31/7036, A61P 11/00

(54) **Antibacterial compositions for the treatment of infections of the upper and lower airways**
Antibakterielle Zusammenhaltung zur Behandlung von Infektion der oberen und untereren Luftwege
Compositions antibactériennes pour le traitement des infections des voise respiratoures supérieures et inférieures

(30) Priority: 13.04.2006 IT MI20060742
(43) Date of publication of application: 24.10.2007
(60) Divisional application: 09166600.8
(73) Proprietor: Rottapharm S.p.A., 20122 Milano (IT)
(72) Inventor: Pattini, Patrizia, 20133 Milano (IT)
(74) Representative: Rambelli, Paolo

(56) References cited:
- US-A1- 2004 009 126
- US-A1- 2006 062 738

## Description

This invention relates to compositions comprising an antibacterial agent and a suitable vehicle, which can be administered by nebulisation.

### Prior art

*Pseudomonas aeruginosa* infection is one of the main unfavourable prognostic factors for patients suffering from cystic fibrosis (CF).

This disease is characterised by a gene defect that causes abnormal functioning of CFTR (cystic fibrosis transmembrane conductance regulator), a protein involved in the transmembrane transport of salts.

As a result of altered ion transport in the cell, cystic fibrosis is manifested by an alteration of the secretions of epithelial cells of bronchi, sinuses, pancreas, intestine, bile ducts and sweat glands.

Patients suffering from cystic fibrosis consequently produce mucus which is very thick and difficult to expectorate. The most serious problems generally affect the bronchi, where mucus viscosity makes it difficult for the bronchial cilia to remove inhaled particles. The mucus therefore fails to perform its main task of facilitating the removal of inhaled particles, thus generating conditions highly favourable to bacterial colonisation as a result of stagnation in the upper and lower airways. In particular, nearly all patients develop *Pseudomonas aeruginosa* infection, which becomes irreversible, and consequently chronic. As a result, CF patients are affected by a self-perpetuating trio of events that lead to an inexorable decline in the respiratory function.

This trio of events is known as obstruction - infection - inflammation. The mucus also protects bacteria against attack by antibiotics, which means that very high doses are needed to perform a bactericidal action.

In the case of aminoglycosides, the family of drugs which are most effective against this germ and Gram-negative bacteria in general, concentrations 25 times higher than the MIC are required to perform a bactericidal action, because concentrations 10 times greater than the MIC are considered only just bacteriostatic.

In order to administer repeated cycles of antibiotic treatment at the high concentrations required by chronic infection, antibiotic-based inhalation solutions for nebulisation in the lower airways only have been used for some time. These formulations enable the antibiotic to be conveyed to the site of the infection, allowing the use of high doses and at the same time reducing systemic exposure.

Bacterial colonisation by *Pseudomonas aeruginosa* and other pathogens takes place by direct contact, especially by inhaling particles of bacteria in suspension in the air. The upper airways play a crucial part in this colonisation process, representing a preferential access route for many germs.

The nose normally produces mucus, which has the function of trapping germs, pollen, dust and all the other particles in the air we breathe, preventing them from reaching the internal respiratory organs.

Extreme dryness of the mucus drastically reduces the efficacy of the nasal secretions, which inexorably become the ideal substrate for bacterial growth.

Before becoming chronic, *Pseudomonas* infection is manifested by a first infection, indicated by a first appearance of bacteria in the sputum; at this stage, a specifically-targeted antibiotic treatment can eradicate the germ and delay chronic infection, but the infection later returns intermittently, until it becomes chronic. At this stage, elimination of the germ from the nasal sinuses performs a significant preventive action against bacterial colonisation of the lower airways.

For this reason, specific antibacterial formulations directed towards these pathogens are highly desirable; such formulations should take account of the physiology of the nasal sinuses and of bronchi sinuses and perform an antibacterial and anti-inflammatory action, thus preventing chronic infection and colonisation of the lower airways due to bacterial migration from the upper airways, reduce the bacteria count once the infection can no longer be eradicated, and at the same time reduce the correlated inflammation.

However, chronic bacterial colonisation, especially by Gram-negative germs, and *Pseudomonas* sp. in particular, is a possible and frequent event even in patients not suffering from CF, especially those with PCD (primary ciliary dyskinesia), severe chronic obstructive pulmonary disease (COPD), or patients with functional alterations of the nasal sinuses who are particularly predisposed to bacterial infection and the correlated inflammatory symptoms. At bronchial level, patients with alterations of the bronchial wall presenting sac-shaped areas (bronchiectasis) are particularly exposed to chronic infection.

Hyaluronic acid is a natural biopolymer belonging to the glycosaminoglycan (GAG) family, and is one of the main components of connective tissue matrix. The lungs, together with the skin and intestine, contain the largest amount of hyaluronic acid. Of the many known properties of hyaluronic acid, perhaps the main one is that it binds and retains a considerable amount of water.

Hyaluronic acid has also been used for its properties in numerous inflammation conditions. For example, its action on joint cartilage is known; hyaluronic acid is used by the intra-articular route to increase the viscous properties of the synovial fluid, and also because it performs a direct anti-inflammatory action, in both acute inflammation models and situations of chronic inflammation. The anti-inflammatory properties of hyaluronic acid are also used in dentistry, plastic surgery and dermatology.

The ability of hyaluronic acid to prevent bronchoconstriction in the stress test when administered by inhalation was also recently described.

The administration of antibiotics by inhalation is normally characterised by good systemic tolerability due to low systemic absorption, but is often accompanied by side effects such as bronchospasm due to local reactivity. This event is observed in some 10-15% of patients, who are consequently obliged to stop taking antibiotics, especially tobramycin.

In aerosol treatment of CF patients, treatment with a hypertonic solution (6-7% NaCl) has recently been suggested in addition to antibiotic treatment, with a view to hydrating the viscous secretions, thus improving their rheology and facilitating their elimination. This treatment has been found to improve the respiratory function.

However, the administration of a hypertonic solution presents considerable problems, especially in terms of tolerability, as its inhalation causes inflammation of the respiratory mucosa, pharyngitis, laryngitis with reddening and local oedema, requiring discontinuance of the treatment, in a large number of patients.

### DESCRIPTION OF THE INVENTION

It has now been found that administration of aminoglycoside or fluoroquinolone antibiotics associated with hyaluronic acid, by inhalation (nasal administration or administration in the upper airways) or bronchial administration, gives better therapeutic results than conventional aerosol formulations and a significantly better local tolerability profile. Hyaluronic acid acts synergically with the antibiotic in both upper and lower airways.

In particular, the invention allows more effective treatment of cystic fibrosis patients, as it eradicates the latent foci of infection in the nasal mucosa, combating colonisation of the lower airways and effectively reducing recurrences of infections of the airways. The invention also allows treatment of patients who have to discontinue antibiotic treatment of the lower airways by inhalation due to bronchial intolerance.

It has also been found that the administration of a hypertonic solution, associated with hyaluronic acid or its salts, leads to better local tolerability, with a reduction in the inflammatory component affecting the mucosa of the airways, a lower risk of discontinuance of the treatment, and better compliance by patients.

Examples of aminoglycosides include tobramycin, gentamicin, amikacin and the like. Tobramycin is particularly preferred.

Examples of fluoroquinolones include ciprofloxacin, norfloxacin, ofloxacin and the like. Ciprofloxacin is particularly preferred.

The hyaluronic acid usable according to the invention will preferably have a low or intermediate molecular weight.

In a first aspect thereof, the invention therefore supplies nasal formulations comprising tobramycin (or another aminoglycoside) associated with hyaluronic acid or its acceptable salts, formulated in a single ampoule or system of delivery by nasal inhalation through a suitable nebuliser.

According to a further aspect, the invention supplies formulations in a disposable ampoule containing tobramycin (or another aminoglycoside) in association with hyaluronic acid for nebulisation in the lower airways through a suitable nebuliser.

According to a further aspect, the invention supplies formulations in a disposable ampoule containing ciprofloxacin or another fluoroquinolone in association with hyaluronic acid for nebulisation in the lower airways through a suitable nebuliser.

According to yet a further aspect, the invention supplies nasal spray formulations comprising ciprofloxacin or another fluoroquinolone, associated with hyaluronic acid or its acceptable salts, formulated in a single ampoule or system of delivery by nasal inhalation through a suitable nebuliser.

According to another aspect, the invention supplies formulations in sterile monodose ampoules of 5-7% hypertonic solution containing hyaluronic acid or a salt or ester thereof with a low molecular weight or an intermediate molecular weight at a concentration of between 0.01% and 1%, and preferably between 0.01% and 0.1%.

These formulations are rendered suitable for administration as aerosols in the lower airways by nebulisation through "Venturi effect" compressors such as Pari boy®, Pari Turbo boy®, Nebula®, etc. and suitable nebulisers, or through a vibration system such as Eflow rapid® or ultrasound nebulisation systems such as I-neb AAD.

The monodose formulations according to the invention will preferably be in the form of sterile aqueous solutions.

The ability to retain water typical of hyaluronic acid, and consequently the possibility of hydrating the mucus and making it easier to eliminate, as well as the possibility of promoting the action of the antibiotic by facilitating its penetration into the mucus, produces a synergic effect in the treatment of the lower and upper airways.

For the administration of aerosols in the lower airways, the solutions can be nebulised through a "Venturi effect" compressor such as Pari boy®, Pari Turbo boy®, Nebula®, etc. and suitable nebulisers, or a vibration system such as Eflow rapid®, ultrasound nebulisation systems, or systems such as I-neb AAD.

Each disposable ampoule typically has a volume of between 2 and 5 ml, and contains between 150 and 300 mg of tobramycin sulphate. Hyaluronic acid, its salts (such as sodium salt) or esters have a molecular weight preferably between 10,000 and 2,000,000 Da, more preferably between 50,000 and 200,000, and are present in a concentration of between 0.01 and 5%. The mean aerodynamic diameter of the particles in the nebulised solutions is preferably between 2 and 5 microns.

The solution has a pH of between 5.5 and 8.0, preferably between pH 6 and 7, and is hypotonic, approximately between 100 and 200 mOsm/kg water with (approx.) 0.25 normal saline, and preferably between 150 and 200 mOsm/kg.

In the case of ciprofloxacin, the disposable ampoules have a volume of between 2 and 5 ml, and contain 250 to 1000 mg of the drug in addition to hyaluronic acid as specified above.

The solutions to be nebulised, described above, can also be used for nebulisation in the upper airways, through a suitable system such as SinuNeb®.

In this case, the doses can be lower: for example, tobramycin sulphate from 50 to 150 mg and ciprofloxacin from 100 to 250 mg.

The nasal solution has a pH of between 5.5 and 7.5, and is preferably isotonic.

Alternatively, the nasal spray solutions are contained in a 10, 15 or 20 ml multispray bottle or a 100-120 ml microdiffuser, able to deliver between 0.5 mg and 25 mg of tobramycin sulphate per nostril on each delivery.

The solution also contains hyaluronic acid, its salts or esters, preferably with a molecular weight of between 50,000 and 2,000,000, in the quantity of between 0.01% and 5% by weight, preferably between 0.05% and 1%, and more preferably between 0.05% and 0.5%. The solution is isotonic or weakly hypotonic, and has a pH of between 5 and 7.

The solution can be distributed between 5 ml monodose bottles containing the same composition as the multidose formulation.

Similar formulations of ciprofloxacin, contained in a 10, 15 or 20 ml multispray bottle or a 100 or 120 ml microdiffuser, are able to deliver between 5 mg and 75 mg of ciprofloxacin per nostril on each delivery.

The solution can be in a 5 ml monodose bottle containing the same composition as the multidose formulation.

The invention is illustrated in greater detail in the examples below.

### Example 1

| A 4 ml disposable ampoule contains: | |
|---|---|
| Tobramycin sulphate | 300 mg |
| Sodium hyaluronate | 0.1 % |
| Solution pH | 6 |
| Osmolarity | 180 mOsm/kg |

Viscosity is adjusted to make the formulation nebulisable at bronchial level through a suitable nebuliser.

### Example 2

| A 3 ml disposable ampoule contains: | |
|---|---|
| Tobramycin sulphate | 300 mg |
| Sodium hyaluronate | 0.05% |
| Solution pH | 6 |
| Osmolarity | 180 mOsm/kg |

Viscosity is adjusted to make the formulation nebulisable at bronchial level through a suitable nebuliser such as Pari LCPLUS, Pari eflow rapid®.

### Example 3

| A 4 ml disposable ampoule contains: | |
|---|---|
| Ciprofloxacin | 300 mg |
| Sodium hyaluronate | 0.5% |
| Solution pH | 6 |
| Osmolarity | 180 mOsm/kg |

Viscosity is adjusted to make the formulation nebulisable at bronchial level through a suitable nebuliser.

### Example 4

| A 4 ml disposable ampoule contains: | |
|---|---|
| Ciprofloxacin | 500 mg |
| Sodium hyaluronate | 0.1 % |
| Solution pH | 6 |
| Osmolarity | 180 mOsm/kg |

Viscosity is adjusted to make the formulation nebulisable at bronchial level through a suitable nebuliser.

### Example 5

| A 3 ml disposable ampoule contains: | |
|---|---|
| Tobramycin sulphate | 100 mg |
| Sodium hyaluronate | 0.1 % |
| Solution pH | 7 |

The solution is made isotonic. Viscosity is adjusted to make the formulation nebulisable at bronchial level through a suitable nebuliser such as SinuNeb®.

### Example 6

| A 3 ml disposable ampoule contains: | |
|---|---|
| Ciprofloxacin | 250 mg |
| Sodium hyaluronate | 0.1% |
| Solution pH | 7 |

The solution is made isotonic.

Viscosity is adjusted to make the formulation nebulisable at bronchial level through a suitable nebuliser such as SinuNeb®.

### Example 7

| 20 ml multidose nasal spray bottle | |
|---|---|
| Contains: | |
| Tobramycin sulphate | 600 mg |
| Sodium hyaluronate | 0.25% by weight |

| Isotonic solution at pH 7 | |
|---|---|
| Each spray delivers 0.15 - 0.20 ml | |
| 2 sprays/nostril b.i.d. | |

### Example 8

| 20 ml multidose nasal spray bottle | |
|---|---|
| Contains: | |
| Tobramycin sulphate | 1200 mg |
| Sodium hyaluronate | 0.20% by weight |
| Isotonic solution at pH 7 | |
| Each spray delivers 0.15 - 0.20 ml | |
| 2 sprays/nostril b.i.d. | |

### Example 9

| 15 ml multidose nasal spray bottle | |
|---|---|
| Tobramycin sulphate | 450 mg |
| Sodium hyaluronate | 0.2% by weight |
| Isotonic solution at pH 7 | |
| Each spray delivers 0.15 - 0.20 ml | |
| 2 sprays/nostril b.i.d. | |

### Example 10

20 ml multidose nasal spray bottle as described in Example 7, containing 750 mg ciprofloxacin.

### Example 11

20 ml multidose nasal spray bottle as described in Example 7, containing 1000 mg ciprofloxacin.

### Example 12

| 100 ml microdiffusion bottle | |
|---|---|
| Contains: | |
| Tobramycin sulphate | 1500 mg |
| Sodium hyaluronate | 0.25% by weight |
| Isotonic solution at pH 7 | |
| Each spray delivers 0.15 - 0.20 ml. | |

### Example 13

| 100 ml microdiffusion bottle | |
|---|---|
| Contains: | |
| Tobramycin sulphate | 750 mg |
| Sodium hyaluronate | 0.5% by weight |
| Isotonic solution at pH 7 | |
| Each spray delivers 0.15 - 0.20 ml | |
| 2 sprays/nostril b.i.d. | |

### Example 14

100 ml microdiffusion bottle as described in Example 12, containing 1500 mg of ciprofloxacin.

### Example 15

100 ml microdiffusion bottle as described in Example 12, containing 750 mg ciprofloxacin.

The viscosity is adjusted to make the formulation nebulisable at bronchial level through a suitable nebuliser.

### Example 16

| 4 ml Disposable ampoule | |
|---|---|
| Sterile solution | |
| NaCl 7% | |
| Sodium hyaluronate | 0.05% |

### Example 17

| 4 ml Disposable ampoule | |
|---|---|
| Sterile solution | |
| NaCl 7% | |
| Sodium hyaluronate | 0.02% |

### Example 18

Kit consisting of 112 ampoules (28 day - treatment)

| | |
|---|---|
| 56 sterile monodose ampoules | 4 ml |
| Tobramycin | 300 mg |
| Sodium hyaluronate | 0.1% |
| Solution at pH 6 | |
| 56 x 4 ml sterile monodose ampoules (28 day - treatment) | |
| Sterile solution | |
| NaCl 7% | |
| Sodium hyaluronate | 0.02% |

## Claims

1. Nebulisable inhalation compositions in form of solutions of aminoglycoside or tluoroquinolone antibiotics, associated with hyaluronic acid.

2. Compositions as claimed in claim 1, administered by the nasal route.

3. Compositions as claimed in claim 1 or 2, in the form of nebulisable monodose ampoules.

4. Compositions as claimed in any one of claims 1 to 3, administrable with a multispray bottle or microdiffuser.

5. Compositions as claimed in any one of claims 1 to 4, including tobramycin, gentamicin or amikacin.

6. Compositions as claimed in claim 4, including tobramycin.

7. Compositions as claimed in any one of claims 1 to 5, including ciprofloxacin, norfloxacin or ofloxacin.

8. Compositions as claimed in claim 7, including ciprofloxacin.

9. Compositions as claimed in any one of claims 1 to 8, also including hyaluronic acid, or a salt or ester thereof, with a low or intermediate molecular weight.

10. Compositions as claimed in any of claims 1 to 9, having a pH of between 5 and 8.0 and an osmolarity of between 100 and 200 mOsm/kg.

11. Compositions as claimed in any one of claims 1 to 10, containing 150 to 300 mg of tobramycin sulphate or 250 to 1000 mg of ciprofloxacin for administration in the upper airways, and 50 to 150 mg of tobramycin sulphate or 100 to 250 mg of ciprofloxacin for nasal administration, in order to dispense an amount of 0.5 mg to 25 mg of tobramycin sulphate or 5 mg to 25 mg of ciprofloxacin per nostril.

12. Compositions as claimed in any one of claims 1 to 11, containing hyaluronic acid or salts or esters thereof, with a molecular weight between 50,000 and 2,000,000 Da, at a concentration between 0.01 and 5%.

13. Compositions of claims 1-12 for use in the treatment of nasal and bronchopulmonary infections.

## Patentansprüche

1. Zerstäubbare Inhalationszusammensetzungen in der Form von Lösungen aus Aminoglykosid- oder Fluoroquinolon-Antibiotika, assoziiert mit Hyaluronsäure.

2. Zusammensetzungen gemäß Anspruch 1, die über die Nasenwege verabreicht werden.

3. Zusammensetzungen gemäß Anspruch 1 oder 2 in der Form von zerstäubbaren Einzeldosis-Ampullen.

4. Zusammensetzungen gemäß einem der Ansprüche 1 bis 3, die mit einer Mehrfachsprühflasche oder einem Mikrozerstäuber verabreichbar sind.

5. Zusammensetzungen gemäß einem der Ansprüche 1 bis 4, die Tobramycin, Gentamicin oder Amikacin enthalten.

6. Zusammensetzungen gemäß Anspruch 4, die Tobramycin enthalten.

7. Zusammensetzungen gemäß einem der Ansprüche 1 bis 5, die Ciprofloxacin, Norfloxacin oder Ofloxacin enthalten.

8. Zusammensetzungen gemäß Anspruch 7, die Ciprofloxacin enthalten.

9. Zusammensetzungen gemäß einem der Ansprüche 1 bis 8, die auch Hyaluronsäure oder ein Salz oder Ester daraus enthalten, mit einem niedrigen oder mittleren Molekulargewicht.

10. Zusammensetzungen gemäß einem der Ansprüche 1 bis 9, die einen pH von zwischen 5 und 8,0 und eine Osmolarität zwischen 100 und 200 mOsm/kg aufweisen.

11. Zusammensetzungen gemäß einem der Ansprüche 1 bis 10, die 150 bis 300 mg Tobramycinsulfat oder 250 bis 1000 mg Cibrofloxacin für die Verabreichung in den oberen Luftwegen enthalten, und 50 bis 150 mg Tobramycinsulfat oder 100 bis 250 mg Cibrofloxacin für die Verabreichung über die Nase, um eine Menge von 0,5 mg bis 25 mg Tobramycinsulfat oder 5 mg bis 25 mg Cibrofloxacin pro Nasenloch zu dosieren.

12. Zusammensetzungen gemäß einem der Ansprüche 1 bis 11, die Hyaluronsäure oder Salze oder Ester daraus enthalten, mit einem Molekulargewicht zwischen 50.000 und 2.000.000 Da mit einer Konzentration zwischen 0,01 und 5%.

13. Zusammensetzungen gemäß einem der Ansprüche 1 bis 12 für die Verwendung bei der Behandlung von nasalen und bronchopulmonalen Infektionen.

## Revendications

1. Compositions pour inhalation nébulisables sous forme de solutions d'antibiotique aminoglycoside ou fluoroquinolone, associé avec l'acide hyaluronique.

2. Compositions selon la revendication 1, administrées par la voie nasale.

3. Compositions selon la revendication 1 ou 2, sous forme d'ampoules monodose nébulisables.

4. Compositions selon l'une quelconque des revendications 1 à 3, administrables avec un flacon multispray ou un microdiffuseur.

5. Compositions selon l'une quelconque des revendications 1 à 4, incluant de la tobramycine, de la gentamicine ou de l'amikacine.

6. Compositions selon la revendication 4, incluant de la tobramycine.

7. Compositions selon l'une quelconque des revendications 1 à 5, incluant de la ciprofloxacine, de la norfloxacine ou de l'ofloxacine.

8. Compositions selon la revendication 7, incluant de la ciprofloxacine.

9. Compositions selon l'une quelconque des revendications 1 à 8, incluant aussi de l'acide hyaluronique, ou un sel ou ester de celui-ci, avec une masse moléculaire basse ou intermédiaire.

10. Compositions selon l'une quelconque des revendications 1 à 9, ayant un pH entre 5 et 8,0 et une osmolarité entre 100 et 200 mOsm/kg.

11. Compositions selon l'une quelconque des revendications 1 à 10, contenant 150 à 300 mg de sulfate de tobramycine ou 250 à 1 000 mg de cyprofloxacine pour l'administration dans les voies aériennes supérieures, et 50 à 150 mg de sulfate de tobramycine ou 100 à 250 mg de ciprofloxacine pour l'administration nasale, pour distribuer une quantité de 0,5 mg à 25 mg de sulfate de tobramycine ou 5 mg à 25 mg de ciprofloxacine par narine.

12. Compositions selon l'une quelconque des revendications 1 à 11, contenant de l'acide hyaluronique ou des sels ou esters de celui-ci, avec une masse moléculaire entre 50 000 et 2 000 000 Da, à une concentration entre 0,01 et 5 %.

13. Compositions selon les revendications 1-12 destinées à être utilisées dans le traitement des infections nasales et broncho-pulmonaires.
